# EUROPEAN PATENT SPECIFICATION

(11) **EP 2 888 398 B1**
(45) Date of publication and mention of the grant of the patent: **30.05.2018**
(21) Application number: 13758965.1
(22) Date of filing: 22.08.2013
(51) Int. Cl.: D06M 13/10, A61K 8/00, C07D 311/30, C11D 9/04, D06M 13/12, D06M 15/03, D06M 16/00, D21H 21/36, C07D 311/32, C08J 7/06, D06M 10/02

(54) **BIOFLAVONOID IMPREGNATED MATERIALS**
BIOFLAVONOIDIMPRÄGNIERTE MATERIALIEN
MATÉRIAUX IMPRÉGNÉS DE BIOFLAVONOÏDES

(30) Priority: 24.08.2012 GB 201215171; 19.10.2012 GB 201218829
(43) Date of publication of application: 01.07.2015
(73) Proprietor: Citrox Biosciences Limited, Bicton Estate Kimbolton Cambridgeshire PE28 0LF (GB)
(72) Inventor: THOMAS, Howard, Cambridge Cambridgeshire PE28 0NJ (GB)
(74) Representative: Avidity IP
(86) International application number: PCT/GB2013/052218
(87) International publication number: WO 2014/030006

(56) References cited:
- EP-A2- 0 350 275
- EP-A2- 1 360 954
- WO-A1-2010/139365
- AU-A4- 2007 100 851
- GB-A- 2 273 291
- JP-A- 2009 041 169
- US-A1- 2011 017 631
- US-A1- 2012 207 806
- US-B1- 6 488 948

## Description

### Field

The present invention relates to bioflavonoid impregnated cellulosic fibrous materials, processes for impregnating the materials and their uses. In particular, the invention relates to bioflavonoid impregnated cellulosic fibrous materials such as paper, paper towels, bamboo fibre and cardboard and articles formed from such materials.

### Background

Cellulosic fibrous materials such as paper are used in a wide variety of applications, ranging from domestic use to commercial use in, for example, hospitals, schools, kitchens and laboratories in the form of, for example, paper towels or face masks or even garments such as bamboo fibre socks.

Some materials would benefit from having antimicrobial properties. These include for example, cardboard, paper, cleaning wipes, paper towels or face masks or even garments.

GB2468836 discloses compositions comprising bioflavonoid compounds and their antibacterial, antifungal and antiviral activity but no suggestion was made that they could be used in impregnating fibres and materials.

### Summary

The present invention relates to cellulosic materials impregnated with a bioflavonoid composition.

According to a first aspect of the invention there is provided a material impregnated with a bioflavonoid composition, the bioflavonoid content of the composition comprising at least naringin and neohesperidin, wherein the naringin and neohesperidin together form at least 50% of the bioflavonoid content.

Especially preferred is when the major part of the bioflavonoid content of the composition comprises naringin and neohesperidin. Preferably, naringin and neohesperidin together form at least 70% wt/wt, for example at least 75% wt/wt, for example 75%-80% wt/wt of the bioflavonoid content of the composition (excluding other biomass).

The bioflavonoid content of the composition may further comprise one or more compounds of Formula (I): 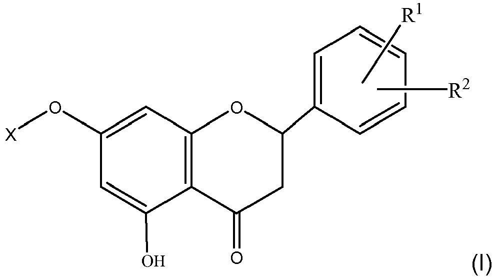 wherein R¹ is a hydroxyl or methoxyl and R² is hydrogen, hydroxyl or methoxyl and X is hydrogen or a saccharide.

A preferred option is when R² is hydrogen and R¹ is in the 3- or 4- position. Another option is when R¹ is 3-hydroxy and R² is 4-methoxyl. Preferably, X is H. More preferably, X is a saccharide.

In preferred cases, X is a disaccharide. Suitable disaccharides include combinations of two monosaccharides, preferably pyranoses, linked by a glycosidic bond, for example rhamnose and glucose, for example L-rhamnose and D-glucose.

Suitable disaccharides can have the structure: 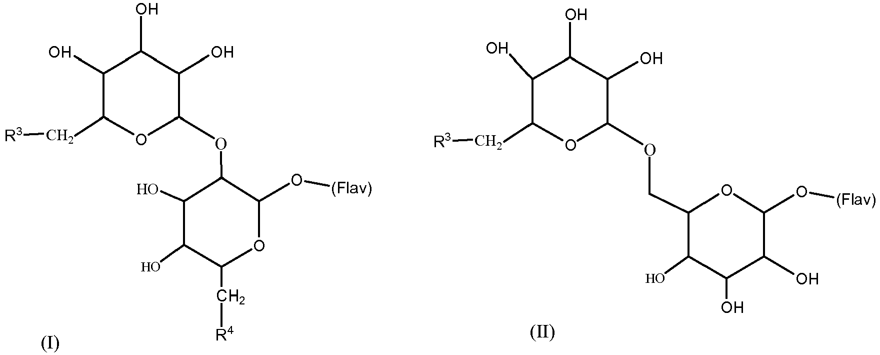 wherein one of R³ and R⁴ is H and the other OH or both are H or both are OH. Preferably R³ is H and R⁴ is OH so that the disaccharide is rutinose.

Favoured aglycones of bioflavonoids for use in this disclosure are the disaccharides 6-O-(alpha-L-rhamnopyranosyl)-beta-D-glucopyranose, also known as rutinose, and 2-O-(alpha-L-rhamnopyra-nosyl)-beta-D-glucopyra-rose.

Suitable compounds of Formula (I) include neoeriocitrin, isonaringin, hesperidin, neodiosmin, naringenin, poncirin and rhiofolin, in addition to naringin and neohesperidin. One of these compounds may be present in addition to naringin and neohesperidin, although a mixture of two or more of these compounds is particularly preferred. A preferred option is wherein the bioflavonoid content of the composition comprises neoeriocitrin, isonaringin, hesperidin, neodiosmin, naringenin, poncirin and rhiofolin.

Such mixtures can be obtained by extraction from bitter oranges and the end product is called *citrus aurantium amara* extract. Particularly preferred are the mixtures of bioflavonoid obtained from the extract of crushed whole immature bitter oranges. The mixtures can also be derived from the starting material comprised of the pith of immature, bitter (blood/red) oranges such as Seville oranges that are classed as 'inedible' and from which the pips, flesh and oily skin have been substantially removed or remain undeveloped.

Suitable mixtures can include 2, 3, 4, 5, 6, 7, 8, 9 or more compounds of Formula (I). A mixture comprising 2, 3, 4, 5, 6, 7, 8, or 9 of the above named bioflavonoids is preferable, for example containing 3, or containing 4, or containing 5, or containing 6, or containing 7, or containing 8, or containing 9 of said bioflavonoids.

It is presently believed that mixtures of such bioflavonoids have advantages over the use of a single bioflavonoid. It is particularly advantageous that extract of bitter oranges is employed without the need for isolating individual bioflavonoids. In an extract from bitter oranges biomass may be associated with up to 40-60% wt/wt, preferably about 55% wt/wt based on the weight of the bioflavonoid content of the composition. The biomass comprises pectins and other sugar derived materials. If it is desired to avoid biomass, other solubilising agents such as dextrines, for example cyclodextrin, may be employed if desired.

A particular advantage of many compositions described herein is that they may employ compounds of natural origin. Thus, for example, it is preferred to employ compounds of Formula (I) from bitter oranges. However synthetically or semi-synthetically obtained compounds may be employed if desired instead of the ones directly extracted from natural sources although this tends to be less favourable in view of cost.

The compositions may further comprise oleuropein. Preferably this is obtained from extraction from the leaf of the olive, for example *Olea europaea.* Such extracts typically contain 5% to 80% wt/wt, more preferably 10% to 70%, for example 20% wt/wt of oleuropein.

The wt/wt ratio of bioflavonoids to oleuropein can be 5:1 to 1:4, preferably 2:1 to 1:2, more preferably 1:2 to 1:1 and even more preferably 3:2. In addition to the bioflavonoid content of the composition, the composition may further comprise one or more fruit acids, for example citric acid, malic acid, and ascorbic acid. One or more of the acids are preferably neutralized with a suitable base, such as a quaternary ammonium base, for example a choline base, such as choline carbonate, bicarbonate or, preferably, hydroxide. More preferably, citric, malic and ascorbic acids are all used in the preparation of the composition, and especially preferred is when these are fully neutralized to provide citrate, malate and/or ascorbate salts. Especially preferred is choline ascorbate.

It has been found that the composition described herein is particularly effective in the presence of one or more organic acids. In one case, the composition further comprises one or more organic acids.

A surprisingly effective organic acid is salicylic acid or its pharmaceutically acceptable salt optionally together with a further organic acid or pharmaceutically acceptable salt.

The salicylic acid may be obtained from willow bark extract. Alternatively, methods for synthesising salicylic acid are known to those skilled in the art.

Sometimes it is preferred that the salicylic acid is in the form of the acid rather than its salt.

Similarly, a further organic acid if present is similarly in the form of the acid rather than its salt. Suitable further organic acids include acids of up to 8 carbon atoms which are monobasic (i.e. one CO₂H group), di-basic or tri-basic acid which optionally contain 1, 2 or 3 hydroxyl groups. Such further organic acid may be one or more of citric acid, malic acid, lactic acid, tartaric acid, fumaric acid and the like.

Such compositions can provide an approximately neutral or acid pH, when used, for example from 3 to 8, more aptly 3.5 to 7, for example 4 to 5.

At present it is preferred to employ salicylic acid and citric acid in the compositions.

Such compositions may include a solubilising agent, for example, salicylic acid such as a dextrin such as cyclodextrin.

The compositions described herein have an extremely favourable safety and environmental profile. As well as showing extremely effective antimicrobial activity, the compositions are also non-toxic, non-corrosive, renewable and completely biodegradable. The compositions disclosed in WO 2012/017186 are the preferred compositions of the present disclosure.

The cellulosic fibrous materials may be composed of paper or cardboard or bamboo fibres. Paper is defined as a material produced from a cellulose pulp which may be derived from wood, rags or grasses. The paper may be in the form of a paper towel, towelette, cloth, wipe or pad. Paper towels have a variety of applications, for example, paper towels are used to dry a person's hands after washing, also known as hand towels. Paper towels or wipes are also used for cleaning purposes to wipe down surfaces in a hospital, laboratory or a kitchen, for example, and can also be known as kitchen roll, kitchen paper or kitchen wipes. Pads are cellulosic fibre sponges and have application in personal hygiene and in medical kits. Wipes are produced as air-laid paper where the fibres are carried and formed to the structure of paper by air.

The paper may be treated with softeners, lotions or added perfume to create a desirable "feel" or texture.

Bamboo materials may be formed of bamboo fibre which is a cellulose fibre extracted or fabricated from natural bamboo. Bamboo is a sustainable crop and, as a natural product derived entirely from plant cellulose, bamboo fibre is biodegradable by microorganisms in soil and also by sunlight. Preferably, the bamboo materials are formed of 100% bamboo fibres although mixtures with other cellulose fibres are also contemplated.

The bamboo may also be in the form of a paper towel, towelette, wipe or pad which may have the same applications as paper towels. The bamboo fibres may also be used as a clothing fabric, optionally in combination with other known fibres, to make garments, such as socks and hospital gowns. For example, socks made from bamboo fibres impregnated with the bioflavonoid compositions described herein can help reduce foot odour. The bioflavonoid impregnated bamboo fibres are activated when they come into contact with moisture from the foot. For hospital gowns, the bioflavonoid composition is activated when the gowns come into contact with, for example, blood or urine.

Fabrics made from bamboo fibres which are impregnated with the bioflavonoid compositions described herein are very useful in hospital or care home environments. For example, the bamboo fabric can be used for bedding sheets, surgical drapes, curtains and the like where it is desirable to use a material with antimicrobial properties.

Paper fibre fabrics can be used instead of the bamboo fibre fabrics described herein; however, the bamboo fibre fabrics are preferred as these fabrics are more durable than paper fibre fabrics.

Paper towels, bamboo towels and the like, may be heated, for example by using a microwave, in order to provide a hot towel. These hot towels may be disposable and/or re-heatable and can be used in restaurants, hotels and on planes.

The bioflavonoid impregnated paper and/or bamboo fibres can also be provided in the form of a face mask, such as a respiratory mask or surgical mask, to provide the user with enhanced protection against inhaling bacteria and viruses or to prevent or reduce the spread of bacteria and viruses. The face masks may be reusable or disposable. Methods of manufacturing face masks are well known in the art.

Bioflavonoid impregnated bamboo and/or paper fibres can be used in the form of single or multi-ply food pads. Such food pads are often found in the bottom of food packaging and can also be referred to as napkins or blankets. The use of these food pads is particularly desirable in food packaging containing food with a short shelf life, for example meat or fruit. The food product, for example, the meat or fruit generally sit on top of the food pad within the packaging. The bioflavonoid impregnated food pad provides a dramatic reduction in the number of bacteria such as *Salmonella, E. coli* and *Campylobacter* which cause foods such as meat and fresh fruit to decay, reducing their shelf life. The bioflavonoid impregnated food pads are particularly suitable in the packaging of meats, including poultry (e.g. chicken or turkey), lamb, beef and pork; fish, including salmon and prawns; and fruits including soft fruits such as blackberries, raspberries, loganberries, strawberries and the like.

Cardboard is heavy duty paper and may include a single thick sheet of paper or more complex configurations such as multiple corrugated and uncorrugated layers which tend to by more durable than regular paper. The cardboard of the present invention will generally be of a depth of less than about 1 cm. The impregnated cardboard can be used in packaging, for example food packaging.

The cellulosic fibrous materials are provided in a dry form and are activated when they are wetted, i.e. when the material comes into contact with moisture, such as a liquid. The liquid may be, for example, water, body fluids, for example sweat, blood or urine, fruit juice, cooking juices and the like. The materials can be wetted before being applied to a surface to be cleaned, for example, by applying water to the material before using on a surface. Alternatively, the materials are activated during use, for example, when drying hands moisture is transferred onto the material or when using the material to wipe down a wet surface.

The materials are provided in a substantially dry form and are preferably dried by heating to constant mass.

Preferably, the amount of bioflavonoid coating impregnated in the material is uniform throughout the material.

The bioflavonoid compositions described herein are biodegradable and can be impregnated into biodegradable materials such as biodegradable paper, bamboo fibres and the like to provide environmentally friendly products.

The bioflavonoid compositions described herein show activity against a wide range of organisms including gram positive bacteria, gram negative bacteria, fungi, virus, protazoans and insect parasites. The compositions may be employed against difficult bacteria such as methicillin resistant *Staphylococcus aureus* (MRSA), *Clostridium difficile* (*C. diff*), *Helicobacter pylori* (*H. pylori*)*,* and vancomycin resistant enterobacteria. The compositions may also be used against norovirus and other pathogens whereby transmission is by contact on air. In particular, the compositions described herein show activity against *E*. *coli, S. aureus, Salmonella, B. subtilis* and *P. aeruginosa.*

According to a second aspect of the invention, there is provided a process for impregnating a cellulosic fibrous material with a bioflavonoid composition according to claim 8. Impregnation is the partial or total saturation of a material, although total saturation is preferred. In particular the material is a thin material. A thin material is defined as having a depth of less than about 1 cm. Impregnation may be after manufacture of the thin material or it may occur during manufacture of the thin material, for example, impregnation of the cellulose fibres before being formed into the material.

If impregnating pre-formed cellulosic fibrous material, the process involves immersing the material, in the bioflavonoid composition to totally or partially saturate the material with the composition. The material may then be rolled, squeezed or wrung to remove any excess of the composition. The material is then dried, either by air drying naturally, oven drying or by mechanical drying. The equipment used to mechanically dry materials will be known to those skilled in the art as will alternative drying methods. The process results in a dry material which can then be packaged as desired and later activated by wetting. Alternatively, the cellulosic fibres used to produce the material may first be immersed in the bioflavonoid composition to totally or partially saturate the fibres with the composition which are then dried either before or after being formed into materials such as paper or cardboard by methods known in the art.

Alternatively, the materials may be impregnated by spraying the bioflavonoid composition onto the materials so that the composition impregnates the outer surface region of the material to achieve at least partial impregnation. Spraying may also be used to impregnate the fibres during manufacture or extraction, before being formed into the materials of the invention.

A particular method for impregnating paper towels is disclosed in Example 3. Fibrous bamboo products may also be impregnated in the same way as disclosed in Example 3.

Preferably, the processes described above provide uniform bioflavonoid impregnation throughout the cellulosic fibrous material. A concentration of between 0.005 and 0.75%, preferably between 0.005 and 0.5%, more preferably between 0.025% and 0.5%, even more preferably between 0.025 and 0.1% of the bioflavonoid composition is used. The compositions described herein are water soluble and water can be used to dilute the bioflavonoid composition to the desired concentration.

According to a third aspect of the invention, there is provided a method of reducing the bacterial load on a surface according to claim 9. The method of reducing the bacterial load on a surface is provided by two mechanisms. Firstly, the kill is achieved by the action of the bioflavonoid compositions and then secondly the contaminants are mechanically removed by the material itself via the action of placing on and wiping the surface, i.e. mechanical wiping.

The surface may be any bioactive surface and could be either a human or non-human surface. For example, a human surface may include the skin on the hands, feet or face. A non-human surface may include any surface of sanitary importance which may carry a contaminant, for example, the surfaces found in schools, bathrooms, kitchens, factories, for example food factories, laboratories, hospitals and the like.

For food contact the Environmental Protection Agency (EPA) requires the active to effect a 5 log reduction of the challenge organism in 30 seconds. Preferably, the materials of the present disclosure effect at least a 5 log reduction of the bacteria load on a surface in 30 seconds.

According to a fourth aspect of the invention, there is provided a packaged product according to claims 10 to 15, wherein the product is formed of a dry cellulosic fibrous material impregnated with a bioflavonoid composition.

The cellulosic product may be individually packaged. Alternatively, the product may be packaged as part of a multi-pack. Known packaging methods and materials may be used to package the products of the present disclosure, for example conventional filmic agents or cardboard boxes.

### Brief Description of the Drawings

In order that the invention may be more fully understood it will now be described, by way of example only, and with reference to the following Figure(s), in which:
Figure 1 is a graph showing the results of the effects of different dilutions of the Citrox BC active dried onto Bounty® brand paper towels on *S*. *aureus* activity.
Figure 2 is a graph showing the results of the effects of different dilutions of the Citrox BC active dried onto Bounty® brand paper towels on *E. coli* activity.

### Detailed Description

The bioflavonoid content may comprise 40-50%, for example about 45% wt/wt of the bioflavonoid composition. A suitable source of a bioflavonoid composition is herein referred to as "HPLC 45" or "Citrox BC" of which about 45% (of the total composition of HPLC 45/Citrox BC) comprises bioflavonoids. The bioflavonoids are in admixture with biomass residues of extraction from bitter oranges, such as pectins, sugars and minor organic acids, which make up the remaining 55%. HPLC 45 is available from Exquim (a company of Grupo Ferrer) as Citrus Bioflavonoid Complex 45% HPLC.

**Table 1: The mixture of bioflavonoids in HPLC 45**

| **Bioflavonoid** | **% bioflavonoid in mixture with biomass** |
|---|---|
| Neoeriocitrin | 1.1 |
| Isonaringin | 1.2 |
| Naringin | 23.4 |
| Hesperidin | 1.4 |
| Neohesperidin | 12.5 |
| Neodiosmin | 1.4 |
| Naringenin | 1.5 |
| Poncirin | 2.0 |
| Other (Rhiofolin) | 0.5 |

### Examples

*Staphylococcus aureus* was chosen as a representative gram positive organism. This organism is found on mammalian skin and is, therefore, shed into the surrounding environment. *E. coli was* chosen as the representative of the gram negative enteric bacteria. This organism is found in the digestive tract of birds, mammals and reptiles. Its presence in the environment signals fecal contamination. *Pseudomonas aeruginosa* was chosen to represent the non-enteric gram-negative bacteria. This genera of bacteria is present in water with related species representing major plant pathogens and human opportunistic pathogens. *Bacillus subtilis* was chosen as the representative gram positive spore-formers. This bacterium is found in soil and water but is also ubiquitous in the environment. This species forms endospores as a survival mechanism. Bacterial endospores are the most resistant form of life on Earth and, therefore, represent an ongoing concern for sanitation, disinfection and sterilisation processes. Endospores represent the "ultimate" challenge for any antimicrobial agent.

### Example 1: Minimum Inhibitory Concentration (MIC) and Minimum Bactericidal Concentration (MBC)

### Procedure

A pure culture of a single microorganism is grown in an appropriate broth. The culture is standardized using standard microbiological techniques to have a concentration of very near 1 million cells per millilitre. The more standard the microbial culture, the more reproducible the test results. The antimicrobial agent is diluted a number of times, 1:1, using sterile diluents. After the antimicrobial agent has been diluted, a volume of the standardised inoculums equal to the volume of the diluted antimicrobial agent is added to each dilution vessel, bringing the microbial concentration to approximately 500,000 cells per millilitre. The inoculated, serially diluted antimicrobial agent is incubated at an appropriate temperature for the test organism for a pre-set period, usually 18 hours. After incubation, the series of dilution vessels is observed for microbial growth, usually indicated by turbidity and/or a pellet of microorganisms in the bottom of the vessel. The last tube in the dilution series that does not demonstrate growth corresponds with the minimum inhibitory concentration (MIC) of the antimicrobial agent.

In order to differentiate between a microbiostatic agent (bacteria are not killed just inhibited) and a microbiocidal agent (bacteria are killed) an MBC test is performed. When a microbiostatic agent is removed or neutralized, previously inhibited bacteria begin to grow again. Each well showing no growth/turbidity in the MIC test is sub-cultured on media that contains no biocide. Any microbial growth resulting from this test indicates that, at that concentration, the active is microbiostatic. If the subculture results in no bacterial regrowth, then, at that concentration, the active is microbiosidal. The range of concentration of Citrox BC active tested was 0.075-0.75%.

### Discussion of Results

The MIC test is an established "screen" for the biostatic (and possibly also biocidal) activity of liquid antimicrobials. It is often used to find the appropriate concentrations of an antimicrobial active to use for further efficacy testing. Performing both the MIC and MBC test will enable one to differentiate between a biocidal or biostatic mode of action. Depending on the concentration of active used and the contact time an active will often demonstrate both biostatic and biocidal modes of action.

The range of Citrox BC active tested was 0.075%-0.75%. For *P*. *aeruginosa,* no MIC value was obtained as all concentrations of the Citrox BC active tested showed no turbidity (Table 2).

MCB testing showed that all concentrations were also bactericidal for *B*. *subtilis,* there was also no MIC value obtained demonstrating that inhibition of growth took place at all concentrations tested. The MBC value obtained for *B*. *subtilis* was 0.315% Citrox BC active. This means that concentrations ranging from 0.075% to 0.315% are bacteristatic and all concentrations of the Citrox BC active greater than or equal to 0.315% are bactericidal.

These results indicate that gram negatives like *P*. *aeruginosa* are more easily killed by the Citrox BC active than the gram positive *B*. *subtilis.*

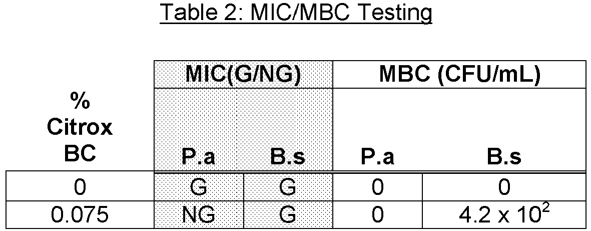

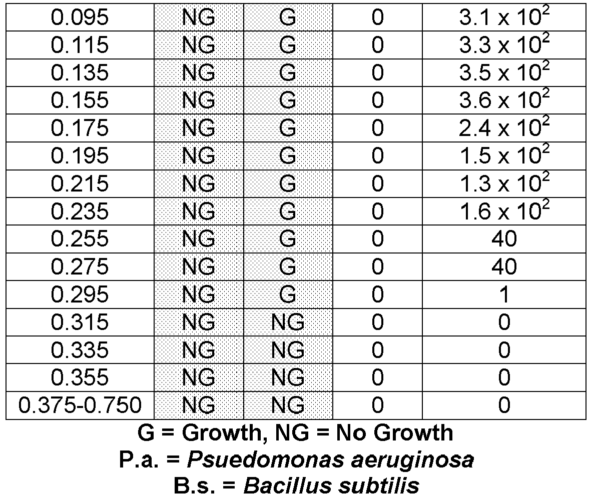

### Example 2: Time Kill Test

### Procedure

All timed kill tests were performed using a standard viable count procedure. Reference NB X34689.

The following neutralising solution was used in all kill tests.
Tween 80 - 3%
Saponin - 3%
Histidine - 0.1 %
Cysteine - 0.1 %

### Rationale

A timed kill test assesses the amount of time it takes to kill a defined population of microorganisms. A wide variety of microorganisms are killed by the Citrox BC active. An important first step in characterising this active for use in an antimicrobial towel is to verify the kill claims. Claims for efficacy are based on the number of bacterial killed within a defined time frame. The most rigorous claims are those made for food contact where the active must affect a 5 log reduction of the challenge organism in 30 seconds.

### Discussion of Results

### Example 2(a): Timed Kill Test: 10 minute Contact Time

Bacterial kill kinetics are affected by bacterial numbers, the concentration of active used and the contact time. In order to determine the most effective range of the Citrox BC active, S. *aureus* was used in a 10 minute kill test to assess the efficacy of various concentrations of the Citrox BC active. A >6.56 log reduction was observed for all concentrations (0.45-0.65%) of the Citrox BC active tested (Table 3).

When *B*. *subtilis* was used as a challenge organism, 0.7% Citrox BC was required to effect a >5 log reduction in 10 minutes (Table 4). Based on previous tests, 0.5% active is the most effective for general use.

**Table 3: Time Kill Test: S. aureus, 10 min.**

| **% Citrox BC** | **CFU/mL** | **Log10 CFU/mL** | **Log Reduction** |
|---|---|---|---|
| 0 | 7.4x10⁶ | 6.86 | 0 |
| 0.45 | <2 | 0.3 | 6.56 |
| 0.5 | <2 | 0.3 | 6.56 |
| 0.55 | <2 | 0.3 | 6.56 |
| 0.6 | <2 | 0.3 | 6.56 |
| 0.65 | <2 | 0.3 | 6.56 |
| 0.65 + neutralizer | 6.6x10⁶ | 6.81 | 0.05 |

**Table 4: Time Kill Test: B. subtilis, 10 min.**

| **% Citrox BC** | **CFU/mL** | **Log10 CFU/mL** | **Log Reduction** |
|---|---|---|---|
| 0 | 1.1x10⁶ | 6.04 | NA |
| 0.5 | 2.9x10⁴ | 4.4 | **1.64** |
| 0.7 | <2 | 0.3 | **5.74** |

### Example 2(b): Timed Kill Test: 30 second Contact Time

Timed kill studies using *E. coli, P. aeruginosa* and *S*. *aureus* were performed using 0.5% Citrox BC active with a contact time of 30 seconds. Log reductions of >6.4 were seen for all organisms (Table 5). This confirms that this active would meet the criteria for use in food contact situations.

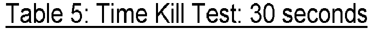

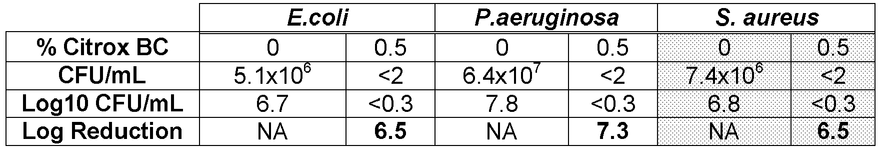

### Example 2(c): Timed Kill Test: Sporicidal Activity

As stated above, the ultimate test for any antimicrobial active is the ability to kill spores. Any chemical or process that kills a bacterial spore is, by definition, a sterilant. In order to assess if the Citrox BC active was sporicidal, a kill test was performed on an actual spore suspension. Citrox BC, over a range 0.5% to 1.5%, was tested over a 1 hour time period. There were some limitations to this test. The spore suspension (*B. subtilis,* ATCC 6633, 6.4x10⁴ CFU/pellet, Microbiologics) in the test was only at ∼2x10⁴ CFU/ml, limiting the log reduction calculation. The lyophilized pellets were found to contain charcoal, a substance known to neutralise the bioflavonoid component of the Citrox BC active. With those limitations, approximately a 2 log reduction in spores was demonstrated. This indicates that the Citrox BC active has definite activity against spores. Spore suspensions at a higher titer without a charcoal additive should be used to investigate this activity further.

### Example 3: Surface Testing using Paper Towel Impregnated with Citrox BC active

### 1) Procedure: Adding Citrox BC to Paper Towel

Bounty® ("Bounty" is a registered trademark of Procter & Gamble) brand paper towels were used to make the dry antimicrobial towels. Bounty® paper towels are a conventional, commercially available paper towel product. Citrox BC active concentrate was diluted to desired concentrations. One paper towel was immersed completely into the diluted active and then wrung out by hand. The towel was dried overnight.

### 2) Procedure: Weight of Citrox BC active dried onto Bounty® brand paper towel

Bounty® brand paper towels were dried to a constant weight in a 54°C oven. Various dilutions of the Citrox BC active were dried onto Bounty® brand paper towels as described above. The towels were dried at room temperature overnight. The treated towels were then dried to a constant weight at 54°C. The weight difference between the untreated and treated towels is presumed to be the weight of the Citrox BC active.

### 3) Procedure: For testing affect of administration of impregnated paper towels to a surface

Using the lab bench top as a representative hard, non-porous surface, a grid was marked off using tape. Cotton-tipped swabs saturated with a broth culture of the challenge organism were used to inoculate the surface and air dried. Paper towels treated with dilutions of the Citrox BC active were wetted and then used to clean the inoculated bench top. The bench top was visibly wet for 3 minutes (contact time) and then allowed to completely air dry. RODAC (Replicate Organism Detection and Counting) plates were used to sample the cleaned surface for surviving bacteria. The plates were incubated overnight at a temperature appropriate to the challenge organism. Colonies were counted and the number used to calculate CFU/cm². Results were calculated by averaging the counts from five 3" x 3" "grid squares".

### 4) Procedure: RODAC sampling

A RODAC plate is used to touch the surface to be sampled after which the plate is incubated at an appropriate temperature. There are nutrients in the media that promote the growth of a variety of microbes. Lecithin and Polysorbate 80 are incorporated in the agar and function as disinfectant/sanitizer neutralisers. The type and number of microorganisms is detected by the appearance of colonies on the surface of the agar medium. Collection of samples from the same area before and after cleaning and treatment with a disinfectant permits the evaluation of sanitary procedures.

### Results

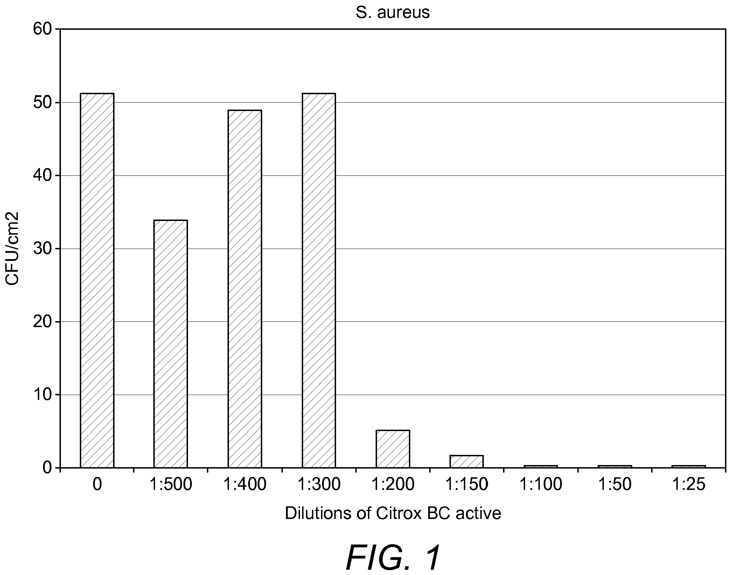

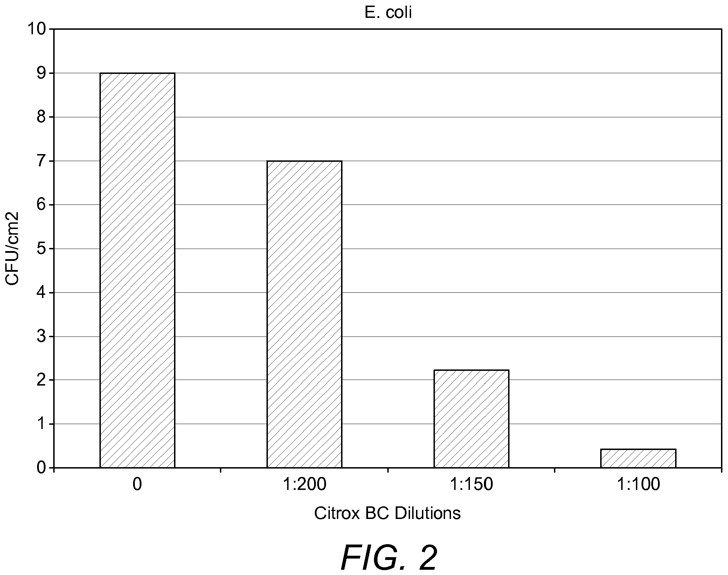

Paper towels wetted with water and containing no Citrox BC active were assessed for the ability to remove bacteria from a contaminated hard surface. The results for this control (i.e. unimpregnated paper towels) are shown by the bar labelled "0" in Figures 1 and 2. Figures 1 and 2 show the results for both *S*. *aureus* and *E. coli.* Paper towels containing dilutions of the Citrox BC active greater than 1:200 were able to reduce the levels of *S*. *aureus* from > 50 CFU/cm² to < 1 CFU/cm². The paper towels containing dilutions of the Citrox BC active greater than 1:200 were able to reduce the levels of *E. coli* from > 7 CFU/cm² to < 1 CFU/cm².

These results show that a dry antimicrobial towel are activated by wetting.

### Discussion of Results

Different dilutions of the Citrox BC active were dried onto Bounty^{®} brand paper towels. These treated towels were used to decontaminate a lab bench heavily inoculated with bacteria. The ability of the treated towels to affect a decrease of contaminants on the lab bench was evaluated using RODAC plates.

A method was developed to assess ability of a paper towel impregnated with the Citrox BC active to reduce bacterial numbers on a contaminated hard surface. RODAC plates are recommended for the detection and enumeration of microorganisms present on surfaces of sanitary importance. RODAC plates are specially constructed so that an agar medium can be overfilled producing a dome-shaped surface that can be pressed on a surface for sampling its microbial content. RODAC plates are used in a variety of programs to establish and monitor cleaning techniques and schedules.

When using a paper towel plus an antimicrobial active, one must keep in mind that removal of bacteria from a contaminated surface occurs by two mechanisms: first is the kill achieved by the action of the antimicrobial active and second is mechanical removal of the contaminants by the paper towel itself.

Lab scale antibacterial towels were used to calculate the weight of the Citrox BC active dried onto the towels. The weight of active present on the towel (Table 6) can be used as a starting point for cost analysis.

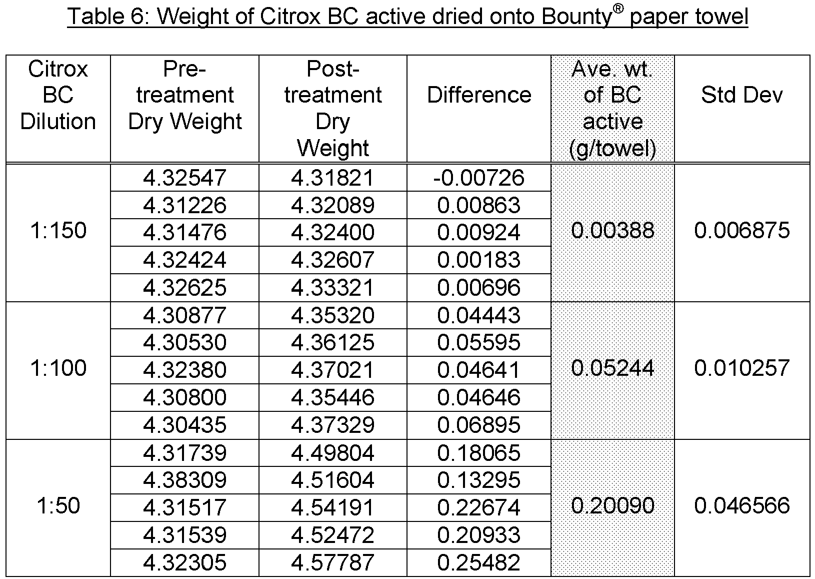

## Claims

1. A dry cellulosic fibrous material impregnated with a bioflavonoid composition, the bioflavonoid content of the composition comprising at least naringin and neohesperidin, wherein the naringin and neohesperidin together form at least 50% of the bioflavonoid content.

2. The material of claim 1, wherein the naringin and neohesperidin together form at least 70% of the bioflavonoid content, at least 75% of the bioflavonoid content or between 75% and 80% of the bioflavonoid content.

3. The material of any preceding claim, wherein the bioflavonoid content of the composition further comprises one or more compounds selected from the group of neoeriocitrin, isonaringin, hesperidin, neodiosmin, naringenin, poncirin and rhiofolin, optionally wherein the bioflavonoid content of the composition comprises neoeriocitrin, isonaringin, hesperidin, neodiosmin, naringenin, poncirin and rhiofolin.

4. The material of any preceding claim, wherein the bioflavonoid composition is uniform throughout the material.

5. The material of any preceding claim, wherein the material is paper or bamboo fibre.

6. The material of claim 5, wherein the paper or bamboo is in the form of a towel or a cloth, in the form of a food pad, in the form of a respiratory mask or in the form of a garment such as a sock or hospital gown.

7. The material of any of claims 1 to 4, wherein the material is cardboard.

8. A process for impregnating a cellulosic fibrous material with a bioflavonoid composition comprising the steps of:
a) immersing or spraying the material or material fibres with the bioflavonoid composition defined in any of claims 1 to 3;
b) mechanically drying the material.

9. A method of reducing the bacterial load on a surface comprising the steps of:
a) wetting the material defined in any of claims 5 or 6 with moisture; and
b) placing the material on a surface and/or wiping the material across the surface; optionally wherein the bacterial load is comprised of bacteria selected from the group of S. *aureus, E. coli, P. aeruginosa, B. subtilis, Salmonella,* MRSA, C. *diff* and *H. pylori.*

10. A packaged product comprising a dry cellulosic fibrous material as defined in any of claims 1 to 4.

11. The product of claim 10, wherein cellulosic fibrous material is as defined in any of claims 5 or 6.

12. The product of either of claims 10 or 11, wherein the product is in the form of a towel or cloth.

13. The product of either of claims 10 or 11, wherein the product is in the form of a food pad.

14. The product of either of claims 10 or 11, wherein the product is in the form of a respiratory mask.

15. The product of either of claims 10 or 11, wherein the product is in the form of a garment such as a sock.

## Patentansprüche

1. Trockenes zellulosisches faserförmiges Material mit einer Bioflavonoid-Zusammensetzung getränkt, wobei der Bioflavonoid-Gehalt der Zusammensetzung wenigstens Naringin und Neohesperidin umfasst, wobei das Naringin und Neohesperidin zusammen wenigstens 50 % des Bioflavonoid-Gehalts ausbilden.

2. Material nach Anspruch 1, wobei das Naringin und das Neohesperidin zusammen wenigstens 70 % des Bioflavonoid-Gehalts , wenigstens 75 % des Bioflavonoid-Gehalts oder zwischen 75 % und 80 % des Bioflavonoid-Gehalts ausbildet.

3. Material nach einem der vorhergehenden Ansprüche, wobei der Bioflavonoid-Gehalt der Zusammensetzung ferner ein oder mehrere Komponenten umfasst, ausgewählt aus der Gruppe aus Neoeriocitrin, Isonaringin, Hesperidin, Neodiosmin, Naringenin, Poncirin und Rhiofolin, optional wobei der Bioflavonoid-Gehalt der Zusammensetzung Neoeriocitrin, Isonaringin, Hesperidin, Neodiosmin, Naringenin, Poncirin und Rhiofolin umfasst.

4. Material nach einem der vorhergehenden Ansprüche, wobei die Bioflavonoid-Zusammensetzung im Material durchgehend einheitlich ist.

5. Material nach einem der vorhergehenden Ansprüche, wobei das Material Papier oder Bambusfaser ist.

6. Material nach Anspruch 5, wobei das Papier oder der Bambus in der Form eines Handtuchs oder eines Tuchs, in der Form einer Lebensmittelunterlage, in der Form einer Atemmaske oder in der Form eines Kleidungsstücks, wie etwa einer Socke oder einem Krankenhaushemd, vorliegt.

7. Material nach einem der Ansprüche 1 bis 4, wobei das Material Pappe ist.

8. Vorgang zum Tränken eines zellulosischen faserförmigen Materials mit einer Bioflavonoid-Zusammensetzung, die folgenden Schritte umfassend:
a) Eintauchen oder Sprühen des Materials oder der Materialfasern mit der Bioflavonoid-Zusammensetzung nach einem der Ansprüche 1 bis 3;
b) mechanisches Trocknen des Materials.

9. Verfahren zum Reduzieren der Bakterienbelastung auf einer Oberfläche, die folgenden Schritte umfassend:
a) Benetzen des Materials nach einem der Ansprüche 5 oder 6 mit Feuchtigkeit; und
b) Anordnen des Materials auf einer Oberfläche und/oder Wischen des Materials über die Oberfläche;
optional wobei die Bakterienbelastung aus Bakterien besteht, ausgewählt aus der Gruppe aus *S*. *aureus, E. coli, P. aeruginosa, B. subtilis, Salmonella,* MRSA, *C. diff* und *H. pylori.*

10. Verpacktes Erzeugnis, ein trockenes zellulosisches faserförmigen Material nach einem der Ansprüche 1 bis 4 umfassend.

11. Erzeugnis nach Anspruch 10, wobei zellulosisches faserförmiges Material nach einem der Ansprüche 5 oder 6 definiert ist.

12. Erzeugnis nach Anspruch 10 oder 11, wobei das Erzeugnis in der Form eines Handtuchs oder eines Tuchs vorliegt.

13. Erzeugnis nach Anspruch 10 oder 11, wobei das Erzeugnis in der Form einer Lebensmittelunterlage vorliegt.

14. Erzeugnis nach Anspruch 10 oder 11, wobei das Erzeugnis in der Form einer Atemmaske vorliegt.

15. Erzeugnis nach Anspruch 10 oder 11, wobei das Erzeugnis in der Form eines Kleidungsstücks, wie etwa einer Socke, vorliegt.

## Revendications

1. Matériau fibreux cellulosique sec imprégné d'une composition de bioflavonoïde, la teneur en bioflavonoïde de la composition comprenant au moins de la naringine et de la néohespéridine, dans lequel la naringine et la néohespéridine forment ensemble au moins 50 % de la teneur en bioflavonoïde.

2. Matériau selon la revendication 1, dans lequel la naringine et la néohespéridine forment ensemble au moins 70 % de la teneur en bioflavonoïde, au moins 75 % de la teneur en bioflavonoïde ou entre 75 % et 80 % de la teneur en bioflavonoïde.

3. Matériau selon une quelconque revendication précédente, dans lequel la teneur en bioflavonoïde de la composition comprend en outre un ou plusieurs composés choisis dans le groupe de la néoériocitrine, l'isonaringine, l'hespéridine, la néodiosmine, la naringénine, la poncirine et la rhiofoline, facultativement dans lequel la teneur en bioflavonoïde de la composition comprend de la néoériocitrine, l'isonaringine, l'hespéridine, la néodiosmine, la naringénine, la poncirine et la rhiofoline.

4. Matériau selon une quelconque revendication précédente, dans lequel la composition de bioflavonoïde est uniforme dans tout le matériau.

5. Matériau selon une quelconque revendication précédente, dans lequel le matériau est de la fibre de papier ou de bambou.

6. Matériau selon la revendication 5, dans lequel le papier ou le bambou se présente sous la forme d'un torchon ou d'un tissu, sous la forme d'une serviette alimentaire, sous la forme d'un masque respiratoire ou sous la forme d'un vêtement tel qu'une chaussette ou une chemise d'hôpital.

7. Matériau selon l'une quelconque des revendications 1 à 4, dans lequel le matériau est du carton.

8. Procédé d'imprégnation d'un matériau fibreux cellulosique avec une composition de bioflavonoïde comprenant les étapes de :
a) mmersion ou pulvérisation du matériau ou des fibres de matériau avec la composition de bioflavonoïde définie dans l'une quelconque des revendications 1 à 3 ;
b) séchage mécanique du matériau.

9. Procédé de réduction de la charge bactérienne sur une surface comprenant les étapes de :
a) mouillage du matériau défini dans l'une quelconque des revendications 5 ou 6 avec de l'humidité ; et
b) placement du matériau sur une surface et/ou essuyage du matériau à travers la surface ; facultativement dans lequel la charge bactérienne est composée de bactéries choisies dans le groupe de *S. aureus, E. coli, P. aeruginosa, B. subtilis, Salmonella,* MRSA, C. *diff* et *H. pylori.*

10. Produit emballé comprenant un matériau fibreux cellulosique sec tel que défini dans l'une quelconque des revendications 1 à 4.

11. Produit selon la revendication 10, dans lequel le matériau fibreux cellulosique est comme défini dans l'une quelconque des revendications 5 ou 6.

12. Produit selon l'une des revendications 10 ou 11, dans lequel le produit se présente sous la forme d'un torchon ou d'un tissu.

13. Produit selon l'une ou l'autre des revendications 10 ou 11, dans lequel le produit se présente sous la forme d'une serviette alimentaire.

14. Produit selon l'une ou l'autre des revendications 10 ou 11, dans lequel le produit se présente sous la forme d'un masque respiratoire.

15. Produit selon l'une ou l'autre des revendications 10 ou 11, dans lequel le produit se présente sous la forme d'un vêtement tel qu'une chaussette.
